(19) Europäisches Patentamt
European Patent Office
Office européen des brevets

(11) Veröffentlichungsnummer: **0 030 342**
**A2**

(12) # EUROPÄISCHE PATENTANMELDUNG

(21) Anmeldenummer: **80107482.4**

(22) Anmeldetag: **29.11.80**

(51) Int. Cl.³: **A 61 F 13/18**, A 61 F 13/00, A 41 B 13/02

(30) Priorität: **10.12.79 GB 7942478**

(43) Veröffentlichungstag der Anmeldung: **17.06.81**
**Patentblatt 81/24**

(84) Benannte Vertragsstaaten: **AT BE CH DE FR IT LI LU NL SE**

(71) Anmelder: **Henkel Kommanditgesellschaft auf Aktien, -Patentabteilung- Postfach 1100 Henkelstrasse 67, D-4000 Düsseldorf 1 (DE)**

(72) Erfinder: **Wiegner, Georg, Breslauerstrasse 35, D-4060 Viersen 11 (DE)**

(54) **Verfahren zur Herstellung einer Hygienevorlage.**

(57) Verfahren zur Herstellung einer Hygienevorlage zum Aufnehmen von Körperflüssigkeit aus einer Flüssigkeitsaustrittsöffnung des Körpers, bestehend aus einer saugfähigen Füllung aus hydrophilen Fasermaterialien mit einer Umhüllung, wobei die Umhüllung aus flüssigkeitsundurchlässigem Verbundmaterial besteht und an der der Flüssigkeitsaustrittsöffnung zuzuwendenden Seite eine definierte flüssigkeitsdurchlässige Saugzone aufweist. Im Kern der Füllung ist eine Einlage mit darin befindlichen hochsaugfähigen Polymeren enthalten. Es wird auf ein Trägermaterial, unter Aussparung der definierten Saugzone, eine Vielzahl flüssigkeitsundurchlässiger Schichten (6) aufgebracht, zum Verbundmaterial vereinigt und in einem gesonderten Arbeitsgang die Umhüllung um die die Einlage enthaltende Füllung herumgefaltet.

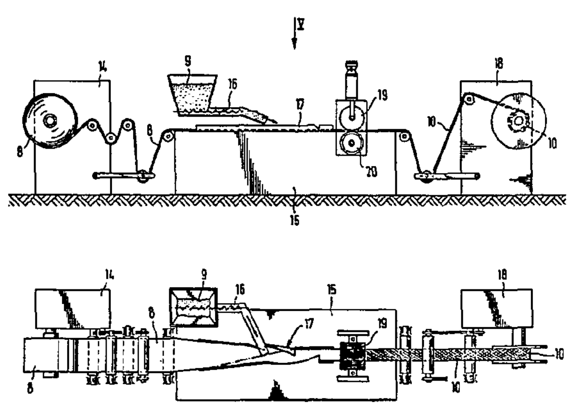

EP 0 030 342 A2

HENKEL KGaA
ZR-FE/Patente
Bor/C

# Patentanmeldung
D 6222 EP

## "Verfahren zur Herstellung einer Hygienevorlage"

Die Erfindung betrifft eine Hygienevorlage zum Aufnehmen von Körperflüssigkeit aus einer Flüssigkeitsaustrittsöffnung des Körpers mit einer saugfähigen Füllung aus hydrophilen Fasermaterialien und mindestens einer Umhüllung und das Verfahren zu deren Herstellung.

Solche Hygienevorlagen werden in verschiedenen Ausführungen verwendet, z.B. in der Baby-Hygiene als Windeln oder Höschen, in der Menstruations-Hygiene als Binden oder Slip-Einlagen, in der Medizin als Wundsekretpackungen oder Urinalvorlagen. Bei diesen Hygienevorlagen ist von großer Bedeutung, daß sie bequem im Tragen, angenehm auf der Haut und nicht zu dick auftragend gestaltet sind und möglichst frei von Geruchsentwicklung. Da es sich um Wegwerfartikel handelt, die relativ häufig verwendet werden, spielen auch der Preis und die Vernichtbarkeit eine Rolle.

Der Verwender ist häufig gezwungen, über mehrere Tage (Binden) oder gar einen langen Zeitraum (Windeln, Slipeinlagen, Urinalvorlagen) Tag und Nacht solche Vorlagen zu tragen. Die Benutzung der Hygienevorlage führt daher häufig zu erheblichen Belastungen, wie Hautirritationen, Geruchsentwicklung oder Beschmutzung der Wäsche und Kleidung. Des weiteren fühlen sich die Verwender oft gehemmt, psychisch belastet durch die Tatsache, daß ihre Umwelt Kenntnis von der Verwendung von Hygienevorlagen erhält, z.B. dadurch, daß wegen der Größe der Vorlage diese durch die Kleidung sichtbar ist oder das Tragen von

...

leichter Kleidung, insbesondere von Badezeug, unmöglich wird. An warmen Sommertagen kommt es häufig zu Schweißbildungen im Bereich der Vorlagen, die sehr lästig sind. Angstzustände können geradezu die Vorstellung auslösen, daß die Vorlage nicht mit Sicherheit die Körperflüssigkeit bindet und es möglicherweise zu einem für jedermann sichtbaren Durchsickern kommt.

Alles in allem ist trotz in der Vergangenheit erzielter erheblicher Fortschritte beim Gestalten von Hygienevorlagen bis heute noch kein umfassend befriedigendes Ergebnis erzielt worden. Ein solches würde voraussetzen, daß ein Produkt folgende Forderungen optimal und zugleich erfüllt:

a) Sicherheit vor Durchsickern;
b) keine wesentlich wahrnehmbare Geruchsbildung;
c) angenehmes Tragen, keine Hautirritationen;
d) kleines Volumen und demgemäß auch bei leichter Kleidung nicht sichtbar zu tragen;
e) wirtschaftlich herzustellen und daher preiswert.

Versuche, vorstehende Anforderungen an eine Hygienevorlage zu erfüllen, führten in der Vergangenheit in bezug auf Forderung a) bei zahlreichen Produkten zu voluminösen Ausführungen, die zwar eine ausreichende Saugfähigkeit aufweisen, aber weder die Forderung d) noch e) erfüllen.

Es sind auch schon Folien auf der körperabgewandten Seite der Vorlage eingesetzt worden, um ein Durchsickern zu verhindern. Dadurch werden jedoch die Forderungen nach angenehmer Tragbarkeit und kleinem Volumen (b und c) nicht erfüllt und der Flüssigkeitskontakt mit der Haut keineswegs ausgeschlossen. Das kommt daher, daß bei außen auf der Umhüllung angebrachten Folien das Durchsickern, z.B. bei Binden oder Windeln, nicht mit Sicher-

...

heit verhütet ist, da sich die Position der Hygienevorlage zwischen den Schenkeln den anatomischen Bedingungen anpaßt und demgemäß die Ränder der Vorlage mehr oder weniger Kontakt zu Wäsche und Kleidung bekommen. Dieser Kontakt wird durch Geh- oder Strampelbewegungen noch verstärkt, so daß es tendenziell zu einem Verrutschen der Folie und damit zu einem Ausquetschen von Flüssigkeit kommt. Darüber hinaus erzeugen außen auf der Umhüllung angebrachte Folien häufig unerwünschte Rascheleffekte.

Zum Verhindern des Kontaktes der Flüssigkeit mit der Haut oder der Kleidung sind Hygienevorlagen mit einer hydrophoben Umhüllung hergestellt worden. Diese Maßnahme bringt jedoch wenig Erfolg, da hydrophobe Umhüllungen keine flüssigkeitsdichte Vorlage ergeben und durch Körperbewegungen die zunächst gespeicherten Flüssigkeiten fast ungehindert ausgequetscht werden. Durch hydrophobe Umhüllungen kann lediglich erreicht werden, daß die Flüssigkeit nicht wie bei hydrophilen Umhüllungen dochtartig um den äußeren Umfang der Hygienevorlage herumgesaugt wird.

Der Erfindung liegt die Aufgabe zugrunde, eine Hygienevorlage eingangs genannter Art zu schaffen, die die vorgenannten Hauptforderungen a) bis e) gleichzeitig und optimal erfüllt, die sowohl in Anwendung als auch in Anbringung bzw. Fixierung am Körper einfach zu handhaben ist und die einfach und billig herzustellen ist.

Diese Aufgabe wird erfindungsgemäß dadurch gelöst, daß die äußere Umhüllung aus flüssigkeitsundurchlässigem Verbundmaterial besteht und an der der Flüssigkeitsaustrittsöffnung zuzuwendenden Seite eine definierte flüssigkeitsdurchlässige Saugzone aufweist und im Kern der

...

Füllung eine Einlage mit darin befindlichen hochsaugfähigen Polymeren enthält.

Daß die neue Hygienevorlage die gestellte Aufgabe in so hervorragender Weise erfüllt und allen Anforderungen gerecht wird, ist im Zusammenwirken aller Merkmale bedingt. Die definierte Saugzone im Bereich der Flüssigkeitsaustrittsöffnung des Körpers läßt die abgesonderte Flüssigkeit genau dort eintreten, wo sie schnell und vollständig adsorbiert werden soll. Diese gezielte Aufnahme wird noch unterstützt durch die Einlage aus hochsaugfähigen Materialien, die geradezu eine Saug- und Dochtwirkung durch die saugfähige Zone hindurch entfaltet und für ein Festhalten der Flüssigkeit im Innern der Hygienevorlage sorgt. Die flüssigkeitsundurchlässige Umhüllung in Form des Verbundmaterials im gesamten übrigen Bereich der Hygienevorlage verhindert andererseits ein Wiederaustreten der Flüssigkeit bei längerem Gebrauch und heftigen Bewegungen. Hinsichtlich der einzelnen Anforderungen ergibt sich:

Die erfindungsgemäße Hygienevorlage bietet zunächst Sicherheit vor Durchsickern (Forderung a). Das wird dadurch erreicht, daß die Vorlage wegen der flüssigkeitsundurchlässigen Ausrüstung der Umhüllung und wegen der hochsaugfähigen Einlage die in den speziell geschaffenen relativ kleinen, definierten Saugzonen aufgesogenen Körperflüssigkeiten wie in einer Art Feuchtetresor festhält und nicht an umgebende, mit der Vorlage in Kontakt stehende Haut- oder Kleidungspartien abgibt.

Die Geruchsbildung (Forderung b) wird bei der neuen Vorlage weitgehend durch den feuchte- und geruchsdichten Abschluß der aufgesogenen Flüssigkeiten im Innern der Hygienevorlage verhindert.

...

Die neue Vorlage ist ferner angenehm im Tragen und bedeutet keine Gefahr von Hautirritationen (Forderung c). Durch das weitgehende Abschirmen von Haut und Wäsche gegenüber einem Flüssigkeitskontakt werden Hautirritationen verhindert. Die angenehm weiche Beschaffenheit des Trägermaterials der äußeren Umhüllung bleibt auch im Verbundmaterial voll erhalten, da die undurchlässige Beschichtung auf der Innenseite der Vorlage angeordnet ist.

Die Vorlage ist ferner klein und kann auch bei leichter Kleidung getragen werden (Forderung d). Da die hochsaugfähige Einlage im Kern der Hygienevorlage zu einem hohen Prozentsatz ausgenutzt wird, kann die Vorlage wesentlich dünner und kleiner als bisher üblich ausgebildet werden, ohne daß die Gefahr eines Durchfeuchtens besteht.

Dabei spielen die besondere Konstruktion und Anordnung der Einlage gemäß weiterer Ausbildung der Erfindung eine große Rolle. Die Einlage besteht bevorzugt aus einem ein- oder mehrfach gefalteten Tissuepapier auf das vor dem Falten hochsaugfähige Polymere in Granulat- oder Faserform aufgebracht sind. Diese hochsaugfähigen Polymere sind als solche vorbekannt. Sie vermögen das Hundertfache ihres Gewichtes ohne Volumenvergrößerung an Flüssigkeit zu binden. Es kommen insbesondere zwei Stoffgruppen dafür in Betracht, nämlich vernetzte Carboxymethylcellulose und Polyacrylate Letztere sind deshalb ganz besonders bevorzugt, weil sie auch für Blut ein ausgezeichnetes Adsorptionsvermögen aufweisen. Die hochsaugfähigen Polymere sind vorzugsweise auf ein ebenfalls gut saugfähiges Material aufgebracht, für das Watte, aber ganz besonders bevorzugt ein Tissueband in Betracht kommt. Dieses ist sehr gut saugfähig und entfaltet damit eine Dochtwirkung

...

und gibt die aufzunehmende Flüssigkeit gut an die hochsaugfähigen Polymere weiter. Die hochsaugfähigen Polymere werden zweckmäßig durch mehrfaches Falten des Tissuepapiers eingeschlossen und in diesem festgehalten.

Besonders vorteilhaft ist es, dem für die hochsaugfähigen Polymere als Trägermaterial bevorzugt geeigneten Tissueband auf einem Kalander ein Waffelmuster zu geben und damit eine Vielzahl kleiner Taschen zu schaffen, in denen das hochsaugfähige Polymere, insbesondere wenn es als Granulat eingesetzt wird, untergebracht ist. Diese Prägung des Tissuebandes kann vor dem Aufbringen der hochsaugfähigen Polymere erfolgen. Bevorzugt ist aber eine Arbeitsweise, bei der die hochsaugfähigen Polymere auf ein Tissueband mindestens der zweifachen Breite der Einlage aufgebracht werden, dann die freien Randbereiche umgefaltet und die hochsaugfähigen Polymere so eingeschlossen werden und erst daran anschließend durch Kalandrieren das Waffelmuster eingeprägt wird. Das Einprägen schafft dann die Taschen, in denen sich das hochsaugfähige Polymere bevorzugt ansammelt, und verbindet gleichzeitig die zwei oder drei Lagen Tissueband, wie sie durch das Umfalten erhalten werden, genügend fest miteinander. Da die hochsaugfähigen Polymere als Fasern oder feines Granulat lose aneinanderliegen und untereinander keine Kapillarität entwickeln, übernimmt das Tissuepapier mit seiner Dochtwirkung die Drainage für die Flüssigkeit zu den Speichertaschen mit hochsaugfähigen Polymeren. Diese wiederum nehmen zwar bis zum Hundertfachen ihres Gewichts an Flüssigkeit auf, ohne daß sich aber ihr Volumen nennenswert vergrößert und ohne daß diese ~~diese~~ Flüssigkeit wieder abgeben - auch nicht bei Quetscheinwirkung.

Die Anordnung dieses die hochsaugfähigen Polymere enthaltenden Tissuebandes im Kern der Vorlage, wie in Figur 6,

...

7 und 8 dargestellt, garantiert den Verbleib der Flüssigkeit im Innern der Vorlage und vermeidet damit Geruchsbildung und Durchfeuchten, und ermöglicht darüber hinaus eine besonders dünne und kleine Ausführung der Hygienevorlage.

Schließlich ist die Hygienevorlage wirtschaftlich herzustellen und damit preiswert (Forderung e). Wegen der geringen Größe und des entsprechend geringen Materialeinsatzes sowie wegen der besonderen Konstruktion der äußeren Umhüllung ist ein wirtschaftliches Herstellen auf handelsüblichen Maschinen möglich. Die neue Vorlage kann daher sehr preisgünstig angeboten werden.

Das die äußere Umhüllung bildende Verbundmaterial hat bevorzugt folgenden Aufbau:
Es besteht außenseitig aus einem flüssigkeitsdurchlässigen Trägermaterial aus Gewebe, Vlies oder Tissue und ist damit ausgesprochen hautfreundlich und angenehm im Tragen. Innenseitig besteht es mit Ausnahme der definierten Saugzone aus einer flüssigkeitsundurchlässigen Schicht, die den Durchtritt der Flüssigkeit nach außen verhindert. Dabei ist es ganz wesentlich, daß das Trägermaterial und die flüssigkeitsundurchlässige Schicht zu einem Verbundmaterial vereinigt sind, also nicht wie bisher lose aneinanderliegen und gegebenenfalls in Randzonen der Hygienevorlage miteinander verbunden sind. Bei den unvermeidlichen Körperbewegungen führte das immer wieder zum Zwischentreten der Flüssigkeit zwischen die einzelnen Lagen und zum Ausquetschen der Flüssigkeit.

Bevorzugt ist auch das Trägermaterial hydrophob ausgerüstet, weil es im Bereich der definierten Saugzone direkt mit der Flüssigkeit in Berührung kommt und diese nicht aufsaugen soll. Hydrophob bedeutet dabei aber lediglich, daß es keine oder nur wenig Flüssigkeit aufnimmt. Das Träger-

...

material läßt aber trotz seiner hydrophoben Ausrüstung unter Körperkontakt die aufzunehmende Flüssigkeit gut hindurchtreten und von der Füllung aufnehmen.

Die innenseitige flüssigkeitsdurchlässige Schicht der äußeren Umhüllung, also das Verbundmaterial, ist vorzugsweise durch Auflaminieren, Aufkalandrieren, Aufextrudieren, Beschichten, Bondieren oder einseitiges Imprägnieren, Aufdrucken oder Aufstreuen erzeugt. Unter Bondieren versteht man dabei das Auftragen eines Bindemittels, beispielsweise in Form von Kunststoff - oder Kautschuklatices. Beim einseitigen Imprägnieren läßt man das Trägermaterial die Imprägnierflüssigkeit nur an der Oberfläche des Imprägnierbades berühren, sodaß keine Durch- und Durch-Imprägnierung erfolgt. Ganz bevorzugt ist seiner Einfachheit und Schnelligkeit wegen der Auftrag der flüssigkeitsundurchlässigen Schicht durch Bedrucken.

Stofflich besteht die flüssigkeitsdurchlässige Schicht bevorzugt aus Kunststoffolien, Thermoplastpulvern, Thermoplastpasten, Heiß- oder Kaltklebstoffe, Polyurethan, Kautschuk, chemischen Bindern, Imprägniermitteln oder zeitlich begrenzt wasserbeständigen Polyvinylalkoholpolymerisaten, wie sie aus Polyvinylacetaten durch ganz bestimmten Verseifungsgrad hergestellt werden. Die zeitlich begrenzte Wasserbeständigkeit, die hiermit erreicht wird, führt dazu, daß so ausgerüstete Hygienevorlagen mit viel Wasser hinweggespült werden können, d.h. toilettierbar sind.

Bewährt haben sich auch farbige Beschichtungen, Imprägnierungen bzw. Bondierungen und Aufdrucke, weil dadurch die undurchlässigen Bereiche von den durchlässigen Bereichen der Umhüllung deutlich abgesetzt sind. Die far-

...

bige Ausgestaltung der flüssigkeitsundurchlässigen Schicht hat den Vorteil, daß dabei auch Aufdrucke wie z.B. Warenzeichen mit angebracht werden können.

Bei der Hygienevorlage zur Aufnahme von Körperflüssigkeit kommt der definierten Saugzone und ihrer Ausgestaltung besondere Bedeutung zu. Erfindungsgemäß ist aus der äußeren Umhüllung aus flüssigkeitsundurchlässigem Material eine Art Fenster - die sogenannte definierte Saugzone ausgespart, indem dieser Bereich von der flüssigkeitsundurchlässigen Beschichtung ausgespart ist und damit dort die Flüssigkeit durchtreten kann. Diese flüssigkeitsdurchlässige Saugzone kann eine der zu beschirmenden Flüssigkeitsaustrittsöffnung angepaßte Form haben. Die Sicherheit gegen ein Durchsickern oder Vorbeilaufen der aufzufangenden Flüssigkeit wird jedoch unter Umständen erhöht, wenn die Saugzone nach allen Seiten gerichtete Saugarme aufweist. Die Saugzone selbst kann zur Verbesserung der Drainage und zur Weiterleitung zur hochsaugfähigen Einlage hin perforiert oder genadelt sein.

Die Einlage der erfindungsgemäßen Hygienevorlage im Inneren der Füllung besteht vorzugsweise aus einem ein- oder mehrfach gefalteten Tissueband, auf das vor dem Falten die hochsaugfähigen Polymere aufgebracht sind, sei es in Faserform oder bevorzugt als Granulat.

Ganz bevorzugt ist dabei eine Ausführungsform für die Einlage, bei der in das ein- oder mehrfach gefaltete Tissueband mit einem Kalander ein Waffelmuster eingeprägt ist, so daß bei hochsaugfähigen Polymeren in Granulatform diese in einer Vielzahl kleiner Taschen gespeichert sind und damit sehr gleichmäßig und auch unverrückbar über die gesamte Länge und Breite der Einlage

...

festgelegt sind. Dieses Einbringen des Waffelmusters kann bereits vor der Zugabe der hochsaugfähigen Polymeren erfolgen. Bevorzugt werden aber die hochsaugfähigen Polymere vorher auf das Tissueband aufgebracht, dieses dann ein-, bevorzugt aber zweimal umgefaltet und erst dann durch Kalandrieren das Waffelmuster eingeprägt. Das hat den Vorteil, daß sich dabei das Granulat in den so gebildeten Taschen sammelt, gleichzeitig aber auch die Lagen untereinander verbunden werden.

Es ist aber auch möglich und durchaus zweckmäßig, die hochsaugfähigen Polymere zusätzlich auf dem Tissueband zu verankern, beispielsweise durch Klebstoff oder durch vorsichtige Naßbehandlung des Tissuebandes. Die dazu erforderliche Flüssigkeit ist äußerst gering im Vergleich zu den großen Mengen Flüssigkeit, die die hochsaugfähigen Polymere aufzunehmen in der Lage sind, so daß ihre Funktionsfähigkeit durch eine solche Naßbehandlung in keiner nennenswerten Weise beeinträchtigt wird.

Daß die hochsaugfähigen Polymere bevorzugt auf ein Tissueband aufgebracht werden, ist darin begründet, daß Tissue eine ganz hervorragende Verteilerwirkung für Flüssigkeiten hat und eine große Dochtwirkung entfaltet und so die Flüssigkeit hervorragend an das hochsaugfähige polymere Material heranträgt.

Der besondere Vorzug dieser erfindungsgemäßen Ausgestaltung der Hygienevorlage, insbesondere der äußeren Umhüllung und der Einlage, besteht zum weiteren in der außerordentlichen Wirtschaftlichkeit der Verarbeitung und Herstellung. Alle bisher bekannten Verfahren und Maschinen zur Herstellung von Hygienevorlagen verarbeiten

...

alle Bestandteile der Vorlage in einem Arbeitsgang auf der Maschine. Insbesondere werden die verschiedenen Lagen der Umhüllung der Füllung von Rollen zugeführt und müssen durch genaues Positionieren, Umlegen, Überlappen, Verkleben etc. um die Hygienevorlage herum angeordnet werden. Dabei tendieren Hüllmaterialien mit relativ hoher Oberflächenglätte zum Verrutschen. Es ist außerdem einleuchtend, daß je mehr einzelne Bestandteile einer Hygienevorlage auf einer Maschine kombiniert werden müssen, desto komplizierter die Maschinenführung und desto aufwendiger die Arbeitsbelastung durch Einfädeln, Rollenwechsel, Lagerhaltung etc. sich darstellt.

Diese Mängel werden durch den erfindungsgemäßen Hüllstoff weitgehend vermieden. Durch die Tatsache, daß die äußere Umhüllung als Verbundmaterial ausgeführt wird, ergibt sich die technische Möglichkeit, dieses Verbundmaterial völlig unabhängig von der eigentlichen Produktionsmaschine für Hygienevorlagen in einer Vorstufe herzustellen.

Diese Vorstufe läßt sich in der Arbeitsweise räumlich und zeitlich unabhängig von der späteren Hauptstufe trennen. So werden bei einem bevorzugten Verfahren nach rationellsten Fertigungstechniken in großen Maschinenbreiten Materialbahnen gefertigt, die je nach unterschiedlicher Vorlagenbreite später bedarfsweise aufgeschnitten werden. Die so aufgeschnittenen Rollen können nun auf der Hygienevorlagen-Maschine mit besonders hohen Fertigungsgeschwindigkeiten verarbeitet werden, da sie

1. durch den Verbund stabiler sind als vergleichbar mehrbahnig zugeführte Materialien. Hierbei ist als üblich Vliesstoff + Tissue + Kunststoffolie anzusehen.

...

Besonders die nur 15 - 20 µ starke Kunststoffolie tendiert durch statische Aufladung, glatte Oberfläche und Instabilität zu Schwierigkeiten in der Verarbeitung. Aufkaschiert auf das Trägermaterial, also als Verbundmaterial, werden diese Schwierigkeiten vermieden,

2. wiederum bedingt durch den Einsatz eines Verbundmaterials als äußere Umhüllung durch geringere Anzahl von Arbeitsstationen, leichtere Bedienung, weniger Rollenwechsel etc. eine höhere Arbeitsintensität erlauben.

Ein ganz bevorzugtes Verfahren zum Herstellen einer erfindungsgemäßen Hygienevorlage besteht deshalb darin, daß auf ein Trägermaterial, dessen Breite ein Vielfaches der Breite einer Umhüllung ist, unter Aussparung der definierten Saugzone eine Vielzahl nebeneinanderliegender flüssigkeitsdurchlässiger Schichten aufgebracht, zum Verbundmaterial vereinigt, dann in die dem Vielfachen entsprechende Anzahl Einzelbahnen von der Breite einer Umhüllung aufgeteilt und in einem gesonderten Arbeitsgang die Umhüllung um die die Einlage enthaltende Füllung herumgefaltet und abschließend in die einzelnen Hygienevorlagen aufgetrennt wird.

Der besondere Vorteil dieses Verfahrens liegt darin, daß breite Bahnen von Trägermaterial in üblichen breiten Beschichtungsanlagen oder Druckwerken in sehr wirtschaftlicher Art und Weise mit einer Vielzahl nebeneinander angeordneter flüssigkeitsundurchlässiger Schichten versehen und zum Verbundmaterial vereinigt werden können, ohne daß die Geschwindigkeit dieses Arbeitsvorganges abhängig ist von der Geschwindigkeit der Vorrichtung zur Herstellung der Hygienevorlage.

...

Ein bevorzugtes Verfahren zur Herstellung der Einlage mit hochsaugfähigen Polymeren besteht darin, daß auf ein Tissueband von mindestens der zweifachen, bevorzugt der dreifachen Breite der Einlage die hochsaugfähigen Polymere in der Breite der Einlage aufgebracht, der bzw. die freien Randbereiche umgefaltet und anschließend durch Kalandrieren ein Waffelmuster eingeprägt und darauf die einzelnen Lagen des Tissuebandes miteinander vereinigt werden.

Anhand der schematischen Darstellung von Ausführungsbeispielen werden weitere Einzelheiten der Erfindung erläutert. Es zeigen:

Fig. 1 die Draufsicht auf eine Hygienevorlage mit mittig gerade verlaufender Saugzone;

Fig. 2 Querschnitt und Draufsicht der Einlage in Form eines dreifach gefalteten hochsaugfähigen Tissuebandes vor dem Prägen;

Fig. 3 eine Ausschnittvergrößerung der Einlage der Fig. 2 nach dem Einprägen des Waffelmusters durch Kalandrieren;

Fig. 4 und 5 das Herstellen der Einlage durch Aufbringen hochsaugfähiger Polymere auf ein Tissueband in Seitenansicht und Draufsicht;

Fig. 6 und 7 Querschnitte durch zwei verschiedene Ausführungsbeispiele der Hygienevorlage;

Fig. 8 ein Längsschnitt durch die Vorlage nach Fig. 1 und 6;

Fig. 9 eine Vorlage in der Draufsicht mit einer längs angeordneten Saugzone mit zickzackförmigen Saugarmen; und

...

Fig. 10 eine Vorlage in der Draufsicht mit einer Saugzone mit sternförmig angeordneten Saugarmen;

Fig. 11 Querschnitt und Draufsicht von Verbundmaterial der äußeren Umhüllung;

Fig. 12 das Verfahren zur Herstellung des Verbundmaterials für die äußere Umhüllung;

Fig. 13 ein Querschnitt durch das Verbundmaterial;

Fig. 14 das Verfahren zur Herstellung der Hygienevorlage.

Die Zeichnung gemäß Fig. 1 zeigt eine Hygienevorlage in der Draufsicht mit gerade und parallel zur Längserstreckung der Hygienevorlage verlaufender Saugzone 1, beiderseits der Saugzone 1 angeordneten flüssigkeitsundurchlässigen Bereichen 2 und an den Längsenden vorgesehenen Verschlußzonen 3.

Fig. 2 zeigt einen Querschnitt durch die Einlage 10 aus einem hochsaugfähigen Tissueband 8, wobei das Tissueband 8 zweimal gefaltet ist, so daß die hochsaugfähigen Polymere 9 einseitig eine doppelte Tissueüberdeckung haben. Die Einlage 10 ist hier noch nicht durch Kalandrieren geprägt und verfestigt.

Fig. 3 zeigt die vergrößerte Draufsicht auf die Einlage 10 mit Darstellung des Waffelmusters und die durch den Waffelkalander bewirkte Verbindung der drei Tissuelagen und die damit verbundene Bildung der Taschen.

Die Fig. 4 und 5 zeigen das Verfahren zur Fertigung der Einlage 10 und das Aufbringen der hochsaugfähigen Polymere 9.

Von einem Abwickelgerät 14 wird Tissueband 8 einem Streu- und Faltaggregat 15 zugeführt und mittels einer Förderschnecke 16 hochsaugfähiges Polymeres 9 auf das Tissueband 8

...

aufgegeben. In der Faltvorrichtung 17 wird dann von rechts und links je ein Drittel der Bahnbreite des Tissuebandes 8 über das mittlere Drittel hinweggefaltet, so daß sich nunmehr die Breite der Einlage 10 ergibt. Dieses zusammengefaltete Einlageband wird jetzt durch die Kalanderwalzen 19, 20 hindurchgeführt, von denen die obere die harte Stahlwalze 19 ist, die das Waffelmuster einprägt, die untere die weiche Kalanderwalze 20. Im Aufwickelgerät 18 wird schließlich die durch Kalandrieren und Prägen in ihren Einzellagen verbundene Einlage 10 als Endlosband aufgewickelt.

Fig. 6 zeigt einen Querschnitt durch eine Hygienevorlage gemäß Fig. 1. Die Saugzone 1 liegt hier auf der Unterseite der Hygienevorlage in dem Bereich, in dem die auf der Innenseite des Trägermaterials 4 der äußeren Umhüllung 5 vorgesehene flüssigkeitsundurchlässige Beschichtung 6 unterbrochen ist. Die Umhüllung 5 ist im wesentlichen mit einer Füllung 7 aus hydrophilem Fasermaterial, nämlich Zellstofffluff, ausgefüllt. Im Kern der Füllung 7 befindet sich die Einlage 10 aus dem mit hochsaugfähigen Polymeren 9 versehenen Tissueband 8. Auf der der Saugzone 1 gegenüberliegenden Seite der Hygienevorlage ist eine aus Klebstoff 11, zum Beispiel einem Hotmelt, und Silikonpapier 12 gebildete Klebenaht vorgesehen.

Die Hygienevorlage gemäß Fig. 7 ist ganz analog zu denjenigen nach Fig. 6 aufgebaut. Der wesentliche Unterschied besteht darin, daß die Füllung 7 aus Zellstoff-Fluff nochmals in ein umhüllendes Tissuepapier 21 eingehüllt ist.

Fig. 8 zeigt den Längsschnitt durch eine Vorlage gemäß Fig. 1 und 6, und zwar durch die Mitte der Hygienevorlage, also durch den Bereich, in dem die definierte Saugzone 1

...

von der Beschichtung 6 ausgespart ist. Das Tissueband 8 ist hier nur einmal um die Schicht mit hochsaugfähigen Polymeren 9 herumgefaltet. Dabei sind im wesentlichen die Bezugszeichen von Fig. 4 verwendet worden.

Die Hygienevorlage gemäß Fig. 9 unterscheidet sich von derjenigen nach Fig. 1 vor allem dadurch, daß die Saugzone 1 nicht gerade Grenzen zu den flüssigkeitsundurchlässigen Bereichen 2 hat, sondern die Form von zickzackmäßig verlaufenden Längslinien 13 aufweist.

Bei der Hygienevorlage gemäß Fig. 10 liegt die Saugzone 1 im wesentlichen zentral innerhalb eines flüssigkeitsundurchlässigen Bereichs 2. Die Saugzone 1 kann dabei sternförmig angeordnete Saugarme 13 besitzen, die sich teilweise in Längsrichtung der Hygienevorlage und teilweise unregelmäßig in Querrichtung ähnlich den Längslinien 13 von Fig. 9 erstrecken. Für diese Ausführungsform empfiehlt sich zum Aufbringen der flüssigkeitsundurchlässigen Beschichtung 6 auf das Trägermaterial 4 und damit die Schaffung der undurchlässigen Bereiche 2, besonders das Verfahren des Aufdruckens.

Fig. 11 zeigt einen Querschnitt durch das Verbundmaterial der äußeren Umhüllung 5 aus Trägermaterial 4 und der Beschichtung 6 sowie die Draufsicht auf einen Laufabschnitt des Verbundmaterials mit den sichtbaren Saugzonen 1.

In Fig. 12 ist das Verfahren der Herstellung des Verbundmaterials für die äußere Umhüllung 5 am Beispiel einer Damenbinde gezeigt. Auf ein hydrophob ausgerüstetes Trägermaterial 4 von der vierfachen Breite der äußeren Umhüllung 5 einer einzelnen Hygienevorlage werden fünf Streifen dünner Polyäthylenfolie von 15 $\mu$ Stärke zwischen Walzen 22 aufkaschiert, wobei die drei inneren Streifen mit 105 mm doppelt

...

so breit sind wie die beiden äußeren mit 52,5 mm Breite. Der Zwischenraum zwischen den Folieenstreifen, der die Saugzone 1 bildet, beträgt 35,5 mm. An beiden Rändern bleibt ein Randstreifen 24 von 20 mm unkaschiert, der später mit dem Randbeschnitt entfernt wird. Durch Längsauftrennen des so hergestllten Verbundmaterials in der Mitte der breiteren aufgebrachten Folienstreifen mittels des Längsschneiders 23 wird dann die breite Bahn in vier Verbundmaterialstreifen von der Breite einer äußeren Umhüllung 5 aufgeteilt und auf eine Rolle gewickelt.

In Fig. 13 ist das Längsauftrennen in Einzelbahnen der äußeren Umhüllung 5 mit punktierten Linien angedeutet.

Fig. 14 zeigt schematisch das Verfahren zur Herstellung der Hygienevorlage. Zwischen zwei Streifen aus Zellstoff-fluff, die miteinander vereinigt die Füllung 7 bilden, wird die Einlage 10 eingeführt. Von unten her wird das Verbundmaterial als äußere Umhüllung 5 zugeführt, und zwar so, daß bei den undurchlässigen Bereichen 2 die Beschichtung 6 nach oben weist, d.h. das Trägermaterial 4 unten liegt. Das Band aus Füllung 7 und Einlage 10 wird an der Trennstelle 26 mit einer nicht dargestellten Schneidvorrichtung in Einzelkissen 25 aufgetrennt, die in einem Abstand von ca. 2 cm hintereinander auf die äußere Umhüllung 5 aufgelegt werden. Die äußere Umhüllung 5 als einheitliches Verbundmaterial wird anschließend um die Kissen 25 herumgefaltet, so daß sich die Ränder, wie in den Fig. 6 und 7 erkennbar, etwas überlappen. Schließlich wird das Silikonpapier 12 zugeführt und abschließend an der Trennstelle 27 in die einzelnen Hygienevorlagen aufgetrennt unter gleichzeitiger Verbindung der vorn und hinten überstehenden Enden der äußeren Umhüllung 5 unter Prägen.

...

Liste der Bezugszeichen

1 = Saugzone
2 = undurchlässiger Bereich
3 = Verschlußzone
4 = Trägermaterial
5 = äußere Umhüllung (Verbundmaterial)
6 = undurchlässige Beschichtung
7 = Füllung
8 = Tissueband
9 = hochsaugfähiges Polymer
10 = Einlage
11 = Klebstoff
12 = Silikonpapier
13 = Saugarme
14 = Abwickelgerät
15 = Streu- und Faltaggregat
16 = Förderschnecke
17 = Faltvorrichtung
18 = Aufwickelgerät
19 = Kalanderwalze als geheizte Stahlwalze
20 = Kalanderwalze als gekühlte Baumwollwalze
21 = umhüllendes Tissuepapier
22 = Walzen für Verbundmaterial
23 = Längsschneider
24 = Randstreifen
25 = Einzelkissen
26 = Trennstelle für Einzelkissen
27 = Trennstelle für Hygienevorlage

## Patentansprüche

1. Verfahren zur Herstellung einer Hygienevorlage zum Aufnehmen von Körperflüssigkeit aus einer Flüssigkeitsaustrittsöffnung des Körpers, bestehend aus einer saugfähigen Füllung aus hydrophilen Fasermaterialien mit mindestens einer Umhüllung, wobei die äußere Umhüllung (5) aus flüssigkeitsundurchlässigem Verbundmaterial besteht und an der der Flüssigkeitsaustrittsöffnung zuzuwendenden Seite eine definierte flüssigkeitsdurchlässige Saugzone (1) aufweist und im Kern der Füllung eine Einlage (8) mit darin befindlichen hochsaugfähigen Polymeren (9) enthält, dadurch gekennzeichnet, daß auf ein Trägermaterial (4), dessen Breite ein Vielfaches der Breite einer Umhüllung (5) ist, unter Aussparung der definierten Saugzonen (1), eine Vielzahl flüssigkeitsundurchlässiger Schichten (6) aufgebracht, zum Verbundmaterial vereinigt, dann in die dem Vielfachen entsprechende Anzahl Einzelbahnen von der Breite einer Umhüllung (5) aufgeteilt und in einem gesonderten Arbeitsgang die Umhüllung (5) um die die Einlage (10) enthaltenden Füllung (7) herumgefaltet und abschließend in die einzelnen Hygienevorlagen aufgetrennt wird.

2. Verfahren zum Herstellen der Einlage (10) für eine Hygienevorlage nach Anspruch 1, dadurch gekennzeichnet, daß auf ein Tissueband (8) von mindestens der zweifachen Breite der Einlage (10) die hochsaugfähigen Polymere (9) in der Breite der Einlage (10) aufgebracht, der bzw. die freien Randbereiche umgefaltet und anschließend durch Kalandrieren ein Waffelmuster eingeprägt und damit die einzelnen Lagen des Tissuebandes (8) untereinander vereinigt werden.

Patentanmeldung
D 6222 EP

HENKEL KGaA
ZR-FE/Patente

0030342

1/10

Fig. 1

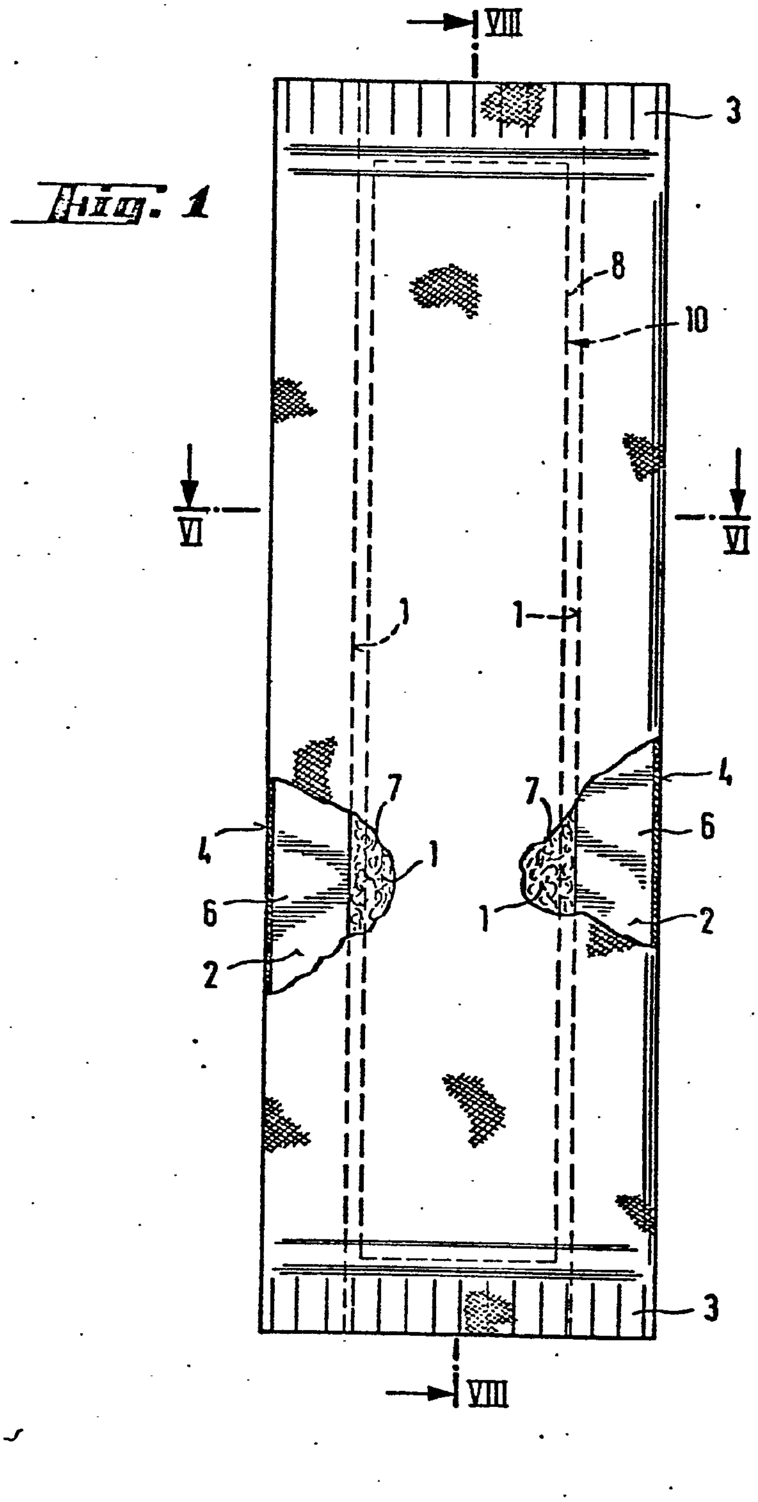

2/10

Fig. 2

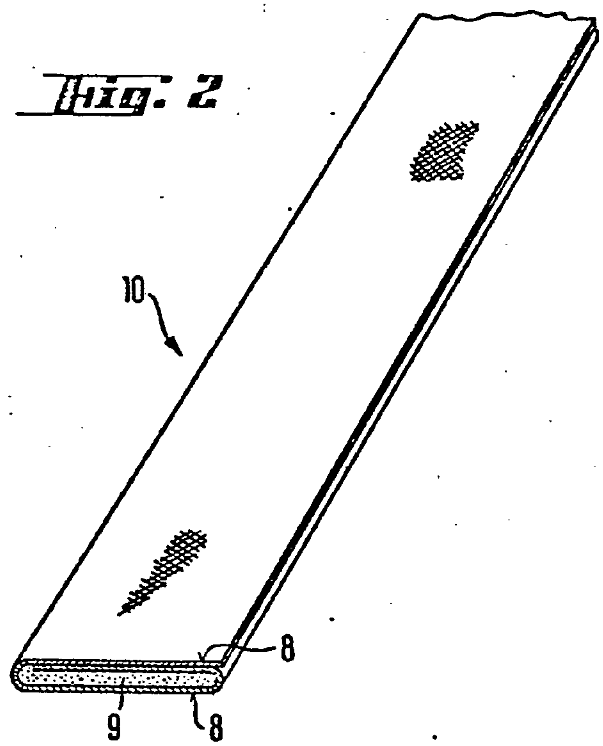


Fig. 3

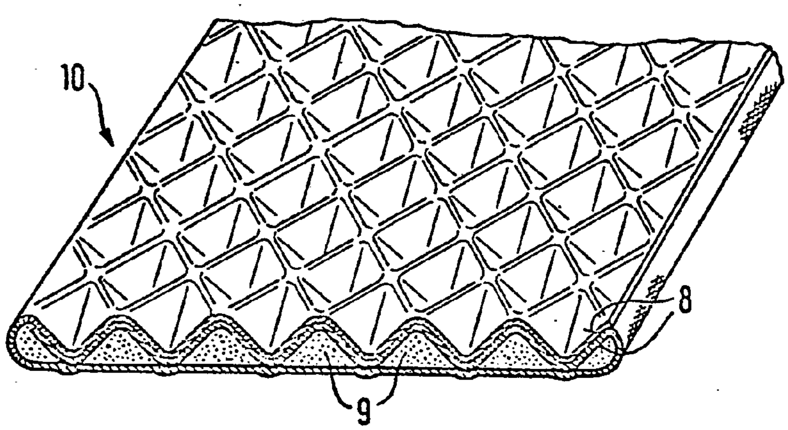

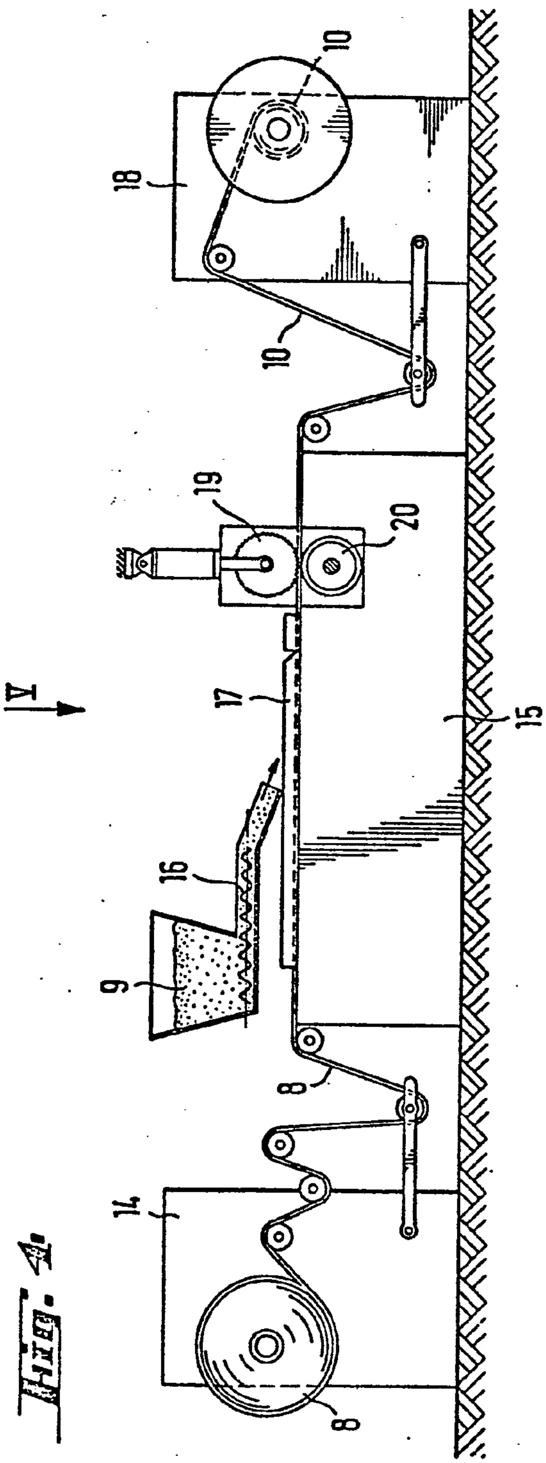


Fig. 4

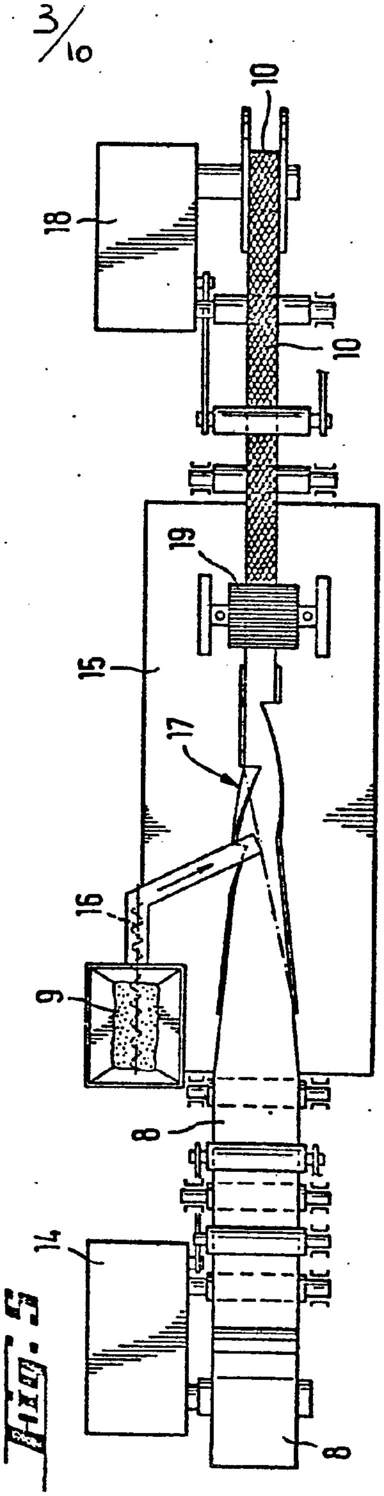


Fig. 5

Fig. 6

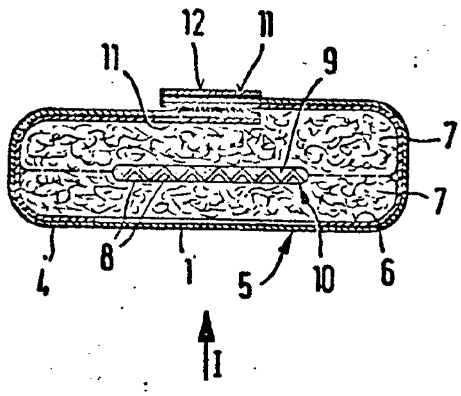


Fig. 8

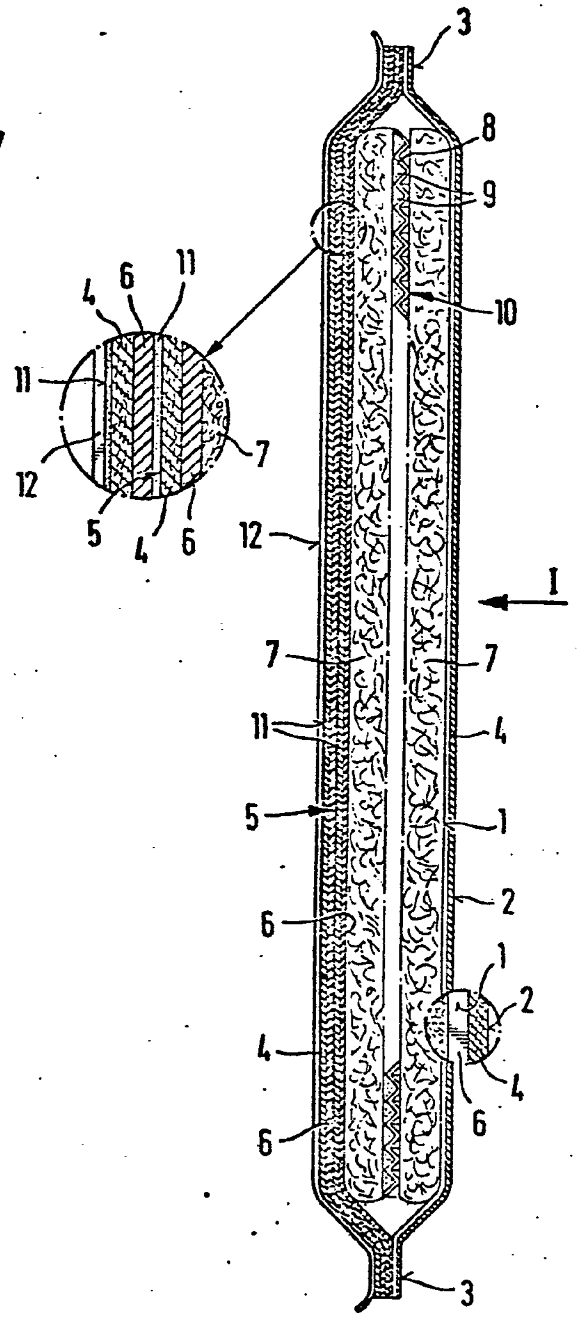


Fig. 7

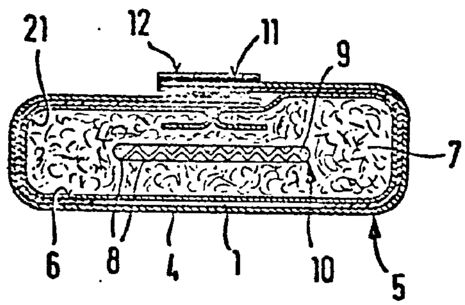

Fig. 9

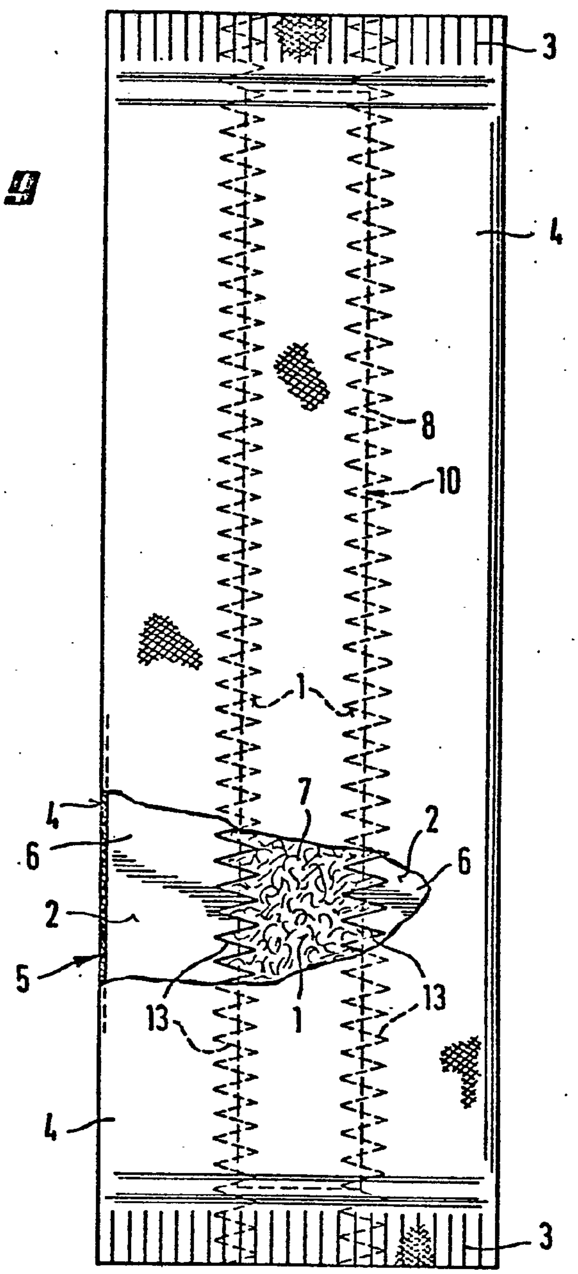

Fig. 1B

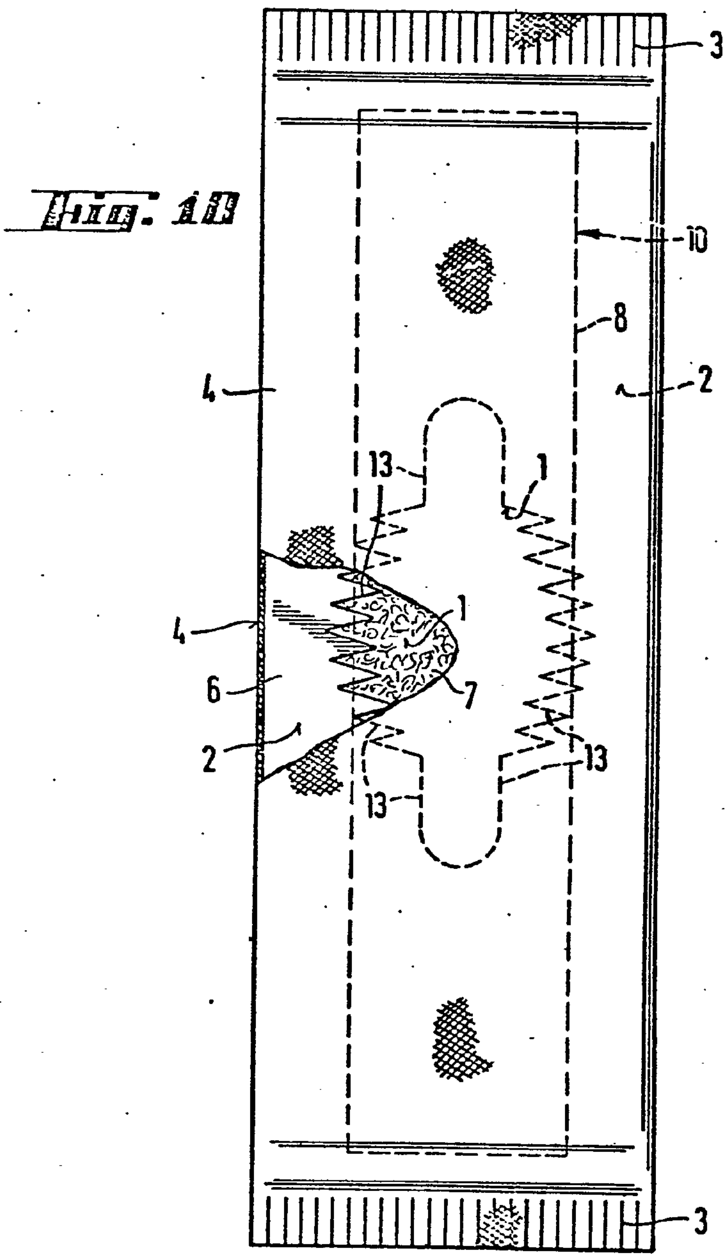

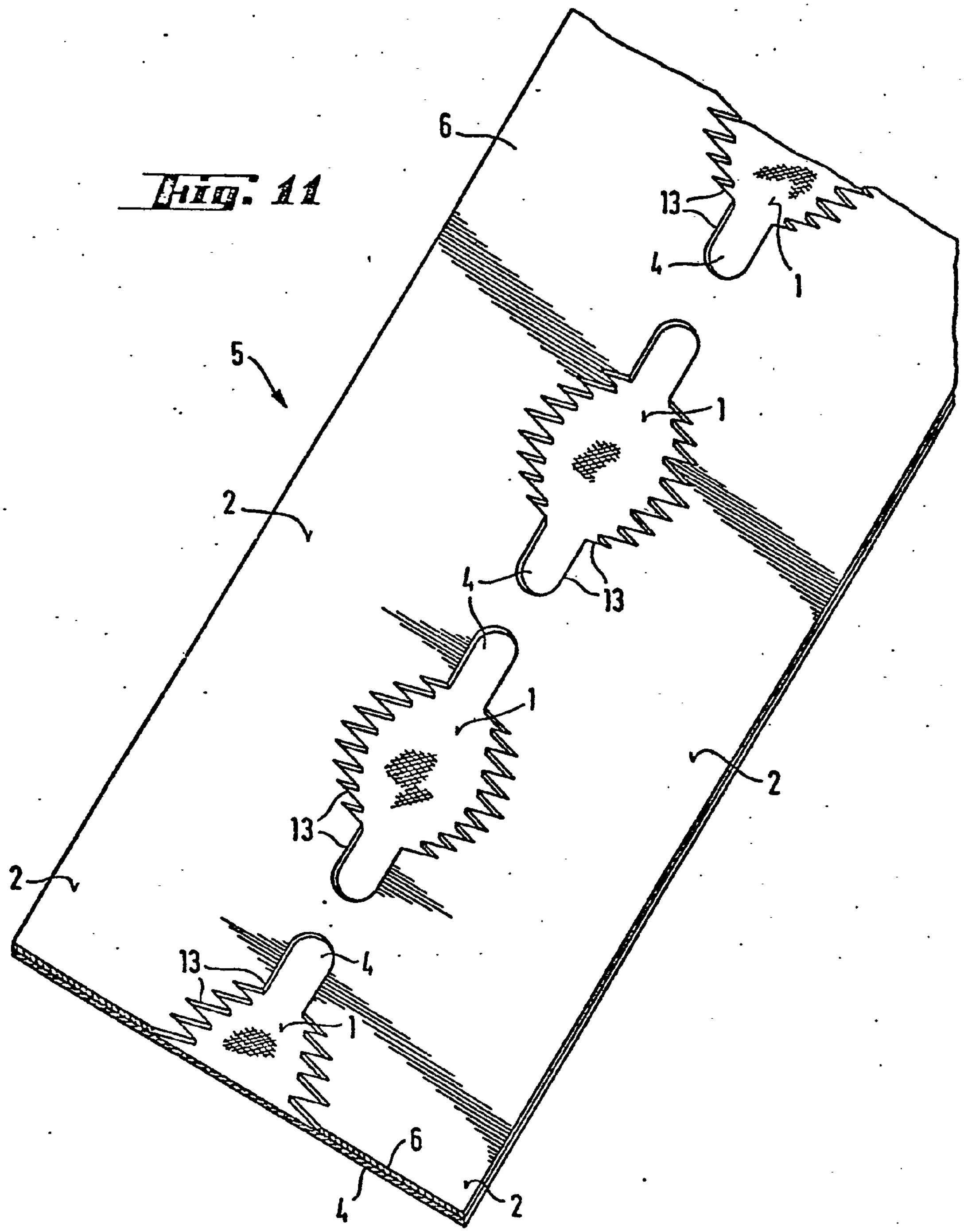


Fig. 11

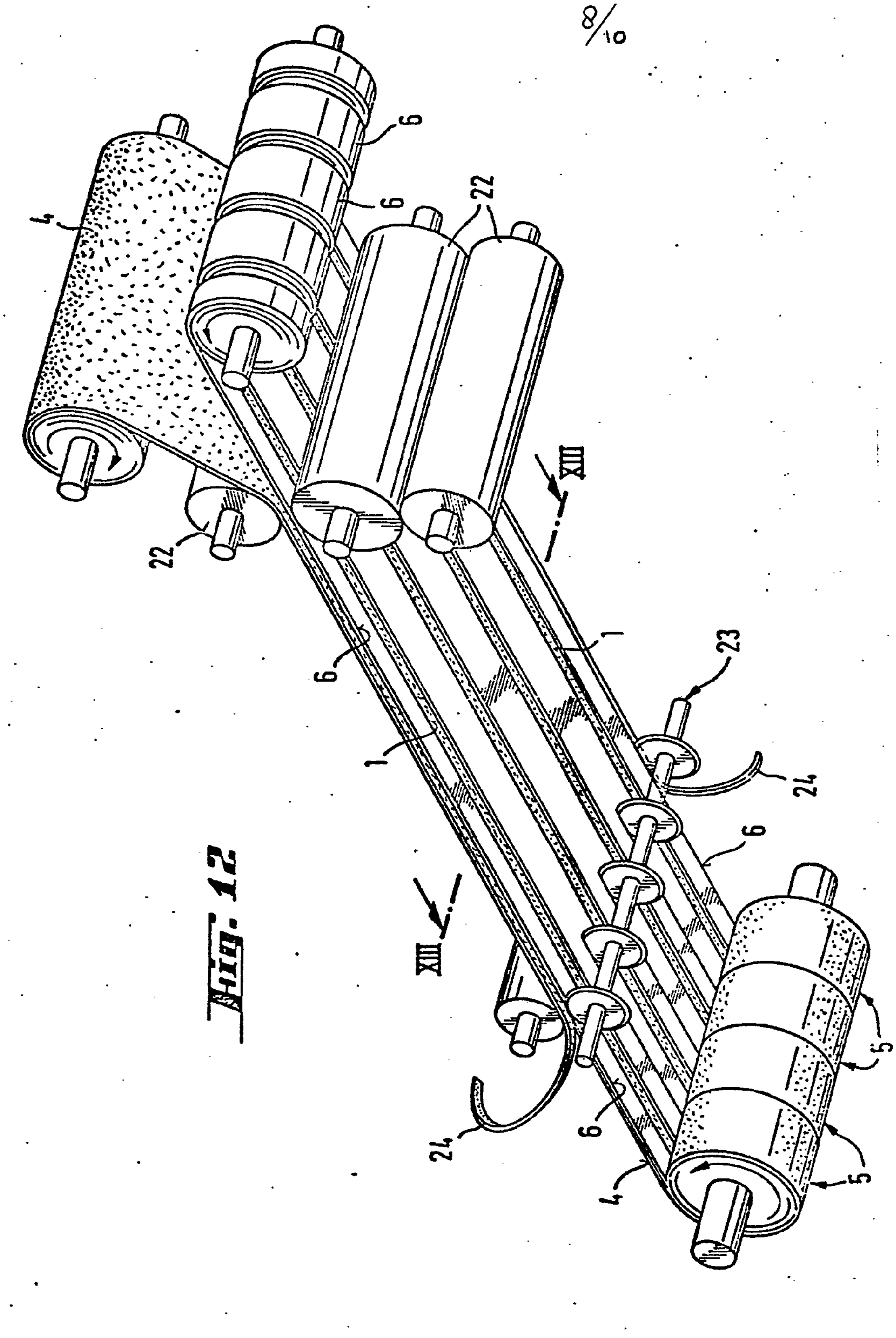


Fig. 12

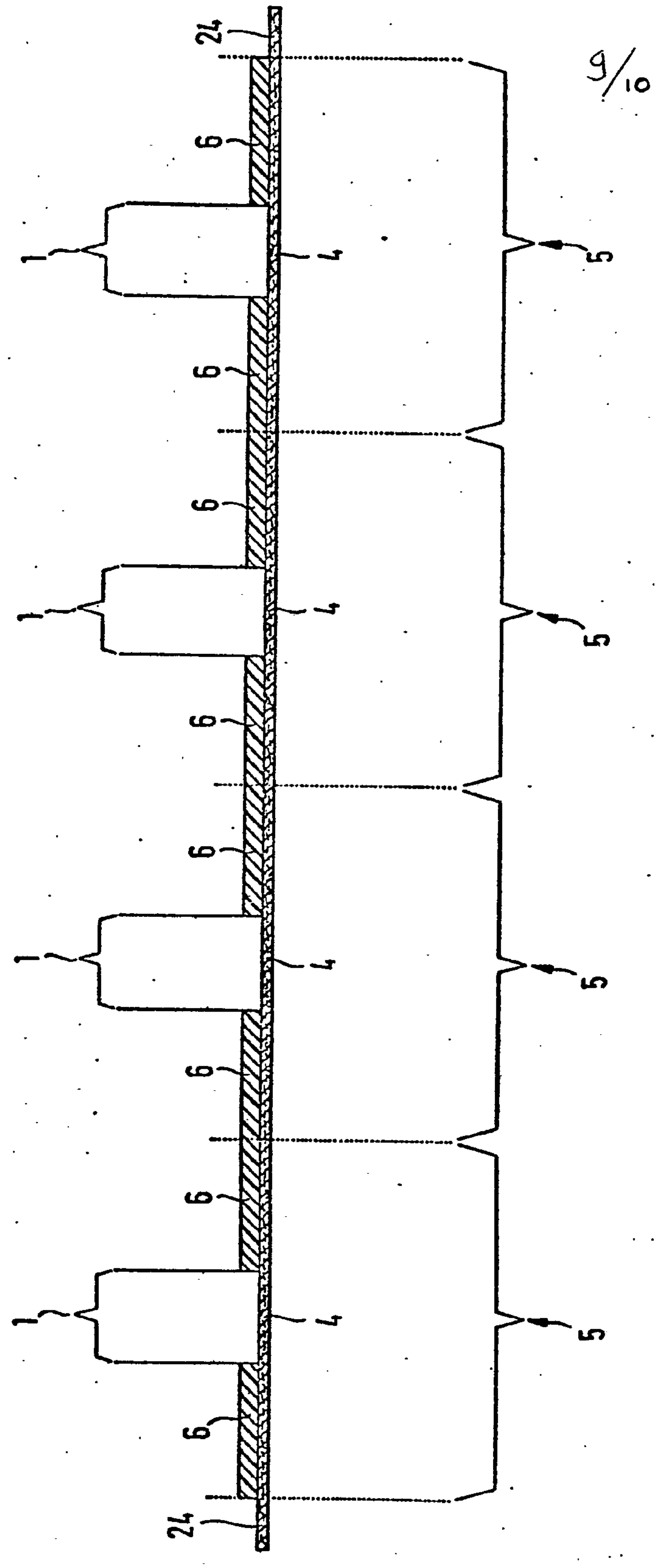


Fig. 13

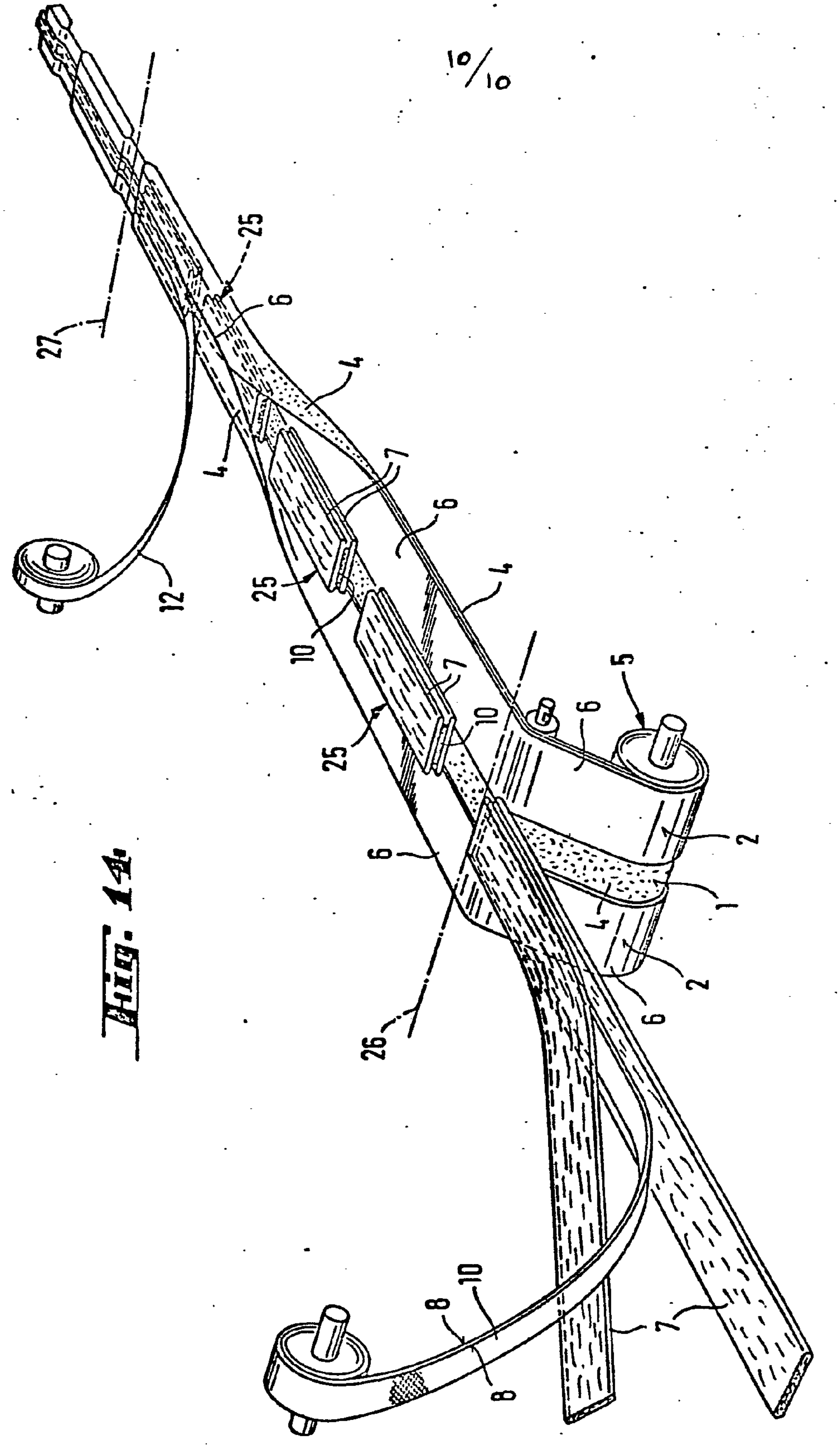

Fig. 14